# EUROPÄISCHE PATENTSCHRIFT

(11) **EP 2 533 740 B1**
(45) Veröffentlichungstag und Bekanntmachung des Hinweises auf die Patenterteilung: **04.11.2015**
(21) Anmeldenummer: 11704209.3
(22) Anmeldetag: 11.02.2011
(51) Int. Cl.: A61F 13/02

(54) **VERBANDSMATERIAL ZUR ÜBERWACHUNG EINER WUNDHEILUNG**
DRESSING MATERIAL FOR MONITORING A WOUND HEALING
MATIÈRE DE PANSEMENT POUR SURVEILLER LA CICATRISATION D'UNE PLAIE

(30) Priorität: 11.02.2010 DE 102010001855
(43) Veröffentlichungstag der Anmeldung: 19.12.2012
(73) Patentinhaber: Fraunhofer-Gesellschaft zur Förderung der angewandten Forschung e.V., 80686 München (DE); Universität Regensburg, 93053 Regensburg (DE)
(72) Erfinder: WOLFBEIS, Otto, S., 93051 Regensburg (DE); MOHR, Gerhard, J., 93407 Regensburg (DE); SZEIMIES, Markus, 93197 Zeitlarn (DE); TRUPP, Sabine, 07318 Saalfeld (DE); STICH, Matthias, 93053 Regensburg (DE)
(74) Vertreter: Stöckeler, Ferdinand
(86) Internationale Anmeldenummer: PCT/EP2011/052057
(87) Internationale Veröffentlichungsnummer: WO 2011/098575

(56) Entgegenhaltungen:
- WO-A1-2011/080427
- US-A1- 2004 043 422

## Beschreibung

Ausführungsbeispiele gemäß der Erfindung beziehen sich auf die Überwachung von Heilungsprozessen von Wunden und insbesondere auf ein Verbandsmaterial zur Überwachung einer Wundheilung, ein Verfahren zum Erkennen einer Änderung einer Eigenschaft einer Wunde und ein Verfahren zum Herstellen eines Verbandsmaterials.

Wunden werden zum Beispiel bei Infektionen alkalisch. Zur Überwachung der Wundheilung muss der Verband, bzw. das Pflaster entfernt werden. Dies ist nicht nur schmerzhaft, sondern kann sich auch ungünstig auf den Heilungsprozess auswirken. Die regelmäßige Kontrolle von Wunden ist aber notwendig, um die Früherkennung von Komplikationen bei der Wundheilung zu gewährleisten.

Das Projekt BIOTEX (siehe "B. Gros, J. Luprano, J. - A. Porchet, R. Rusconi, A. De Sousa, A. Ridolfi, J. Solá I Caros, "CSEM Scientific & Technical Report", 2007, 87") befasst sich mit sogenannten Bio-Sensor-Textilien. Die Erkennung verschiedener Bioanalyte erfolgt dabei über Messungen der Impedanz, Kapazität oder optische Nachweismethoden, wobei die Sensoreinheit in Textilien integriert wird. Die Auswertung der Ergebnisse wird dann in einem transportabeln elektronischen Auslesegerät angezeigt.

Diamond et al. (siehe "D. Morris, S. Coyle, Y. Wu, K.T. Lau, G. Wallace, D. Diamond, Sensors and Actuators B, 139, 2009, 231") beschrieben Bio-Sensor-Textilien zur Messung des pH-Wertes von Schweiß basierend auf einer chemischen oder Indikator-Reaktion. Die Materialien transportieren aufgrund ihrer Beschaffenheit ausreichend Probenvolumen (Schweiß). Die Bestimmung erfolgt durch optische Detektion der Farbänderung eines pH-sensitiven Farbstoffs. Dazu wird das pH-sensitive Bromkresolrot auf die Textile aufgebracht und der Farbumschlag von gelb (pH = 4) nach blau (pH = 7) mittels Emitter-Detektor-LEDs ausgelesen.

Ein Patent (siehe Johnson & Johnson Medical Ltd., Edinburgh, GB, "Patentschrift DE 698 10 366T2", 1997) der Firma Johnson & Johnson beschreibt ein "Verfahren zum Bereitstellen einer Aufzeichnung, welche den Zustand von unterschiedlichen Bereichen einer Wunde zeigt, welches den Schritt eines Aufbringens eines diagnostischen Blatts auf die Oberfläche der Wunde umfasst, dadurch gekennzeichnet, dass das diagnostische Blatt selektiv reaktiv über wenigstens einen Teil seiner Fläche mit einen oder mehrere immunologische oder andere Bindungspartner für das eine oder die mehreren Moleküle umfasst". Der Zustand einer Wunde kann mit der beschriebenen Erfindung anhand mehrerer Faktoren bestimmt werden. Verschiedene Enzyme, die in der Wundflüssigkeit vorhanden sind und auch pH-Wert-Änderungen können durch Reaktionen mit chemilumineszenten, chromogenen und fluorogenen Substraten angezeigt werden. Die Beschreibung erklärt das "diagnostische Blatt" als "reaktive Blattmaterialien zur Aufzeichnung des Zustands einer Wunde". Zur Durchführung der Diagnostik muss also der Verband entfernt werden, gegebenenfalls muss Probenvolumen angesammelt werden (z. B. über eine absorptionsfähige Schicht), zur Auswertung muss dann das "diagnostische Blatt" auf die Wunde, bzw. auf die absorptionsfähige Schicht aufgebracht werden.

Aus der US 5181905 ist ein Wundverband bekannt, der einen Indikator aufweist, der auf Temperaturänderungen anspricht. Aus der US 4813942 ist ein Wundverband bekannt, der Mittel zum Überwachen des pH-Werts aufweist. Aus der WO99/12581 ist ein Wundverband bekannt, der eine Indikatorschicht aufweist, die auf Wasser anspricht. Aus der GB2408330A ist es bekannt, einen Film, vorzugsweise aus Hydrogel, der seine Farbe abhängig vom pH-Wert ändert, zu verwenden, um den pH-Wert um eine Wunde zu beobachten. In der US2008/0108925A1 ist ein Wundverband bekannt, bei dem ein Farbstoff oder ein Biozid, in oder hinter einer Gelatine-Barriere angeordnet ist, um auf Metalloproteinasen anzusprechen, um den Zustand einer Wunde anzuzeigen.

Weitere Verfahren zum Nachweis von Analyten oder Mikroben in Körperflüssigkeiten werden in den US-2004043422 und in den WO-2011080427 offenbart.

Die Aufgabe der vorliegenden Erfindung ist es, ein verbessertes Konzept zur Überwachung einer Wundheilung zu schaffen, die es medizinischem Personal, nicht-medizinischem Personal oder dem Patienten selbst erlaubt, den Heilungsprozess einer Wunde möglichst einfach zu überwachen.

Diese Aufgabe wird durch ein Verbandsmaterial gemäß Anspruch 1, ein Verfahren zum Erkennen einer Änderung einer Eigenschaft einer Wunde gemäß Anspruch 14 oder einem Verfahren zum Herstellen eines Verbandsmaterials gemäß Anspruch 15 gelöst.

Ein Ausführungsbeispiel gemäß der Erfindung schafft ein Verbandsmaterial zur Überwachung einer Wundheilung. Das Verbandsmaterial umfasst ein Verbandsgrundmaterial und ein Kontrollelement. Das Kontrollelement ist in oder auf dem Verbandsgrundmaterial angeordnet, so dass eine Kontrollfläche des Kontrollelements sichtbar ist und eine flüssige oder gasförmige Absonderung einer Wunde mit dem Kontrollelement in Kontakt ist, wenn das Verbandsmaterial auf die Wunde aufgebracht ist. Des Weiteren ist das Kontrollelement ausgelegt, um bei einer Änderung einer Eigenschaft der Wunde, basierend auf einer chemischen Reaktion eine sichtbare Änderung der Kontrollfläche hervorzurufen, um die Überwachung der Wundheilung zu ermöglichen. Das Kontrollelement weist zumindest einen Rezeptor auf, der ausgelegt ist, um selektiv auf einen oder mehrere Analyten anzusprechen, der oder die aus der Gruppe ausgewählt sind, die Amine, Alkohole, Thiole, Aldehyde, Ketone, Diole, Wasserstoffperoxid, Stickoxid, Aminosäuren, Carbonsäuren, Schwefelwasserstoff und Mercaptane enthält.

Ausführungsbeispiele gemäß der Erfindung basieren auf dem Kerngedanken, ein Kontrollelement, das auf eine Veränderung einer Eigenschaft der Wunde selektiv ansprechend auf bestimmte Analyten mit einer sichtbaren Änderung einer Kontrollfläche reagiert, so anzuordnen, dass diese Änderung beobachtet werden kann, ohne das Verbandsmaterial von der Wunde zu entfernen.

Bei Ausführungsbeispielen der Erfindung weist das Kontrollelement einen oder mehrere Farbstoffe auf, die mit Rezeptorfunktionen zur selektiven Erkennung von Analytmolekülen ausgestattet sind. Die Farbstoffe bzw. die Rezeptorfunktion reagiert auf das Vorliegen entsprechender Analytmoleküle selektiv mit einer sichtbaren Änderung, beispielsweise einer Farbänderung oder einer Helligkeitsänderung. Bei Ausführungsbeispielen der Erfindung kann die Eigenschaftsänderung des Kontrollelements in einer Fluoreszenz und/oder Absorption stattfinden.

Die sichtbare Änderung kann detektiert werde, beispielsweise visuell wahrgenommen werden und/oder spektroskopiert werden.

Dadurch kann einerseits dem Pflegepersonal oder dem Patienten Zeit und auch Geld gespart werden, da das Verbandsmaterial nicht entfernt werden muss, um die Wunde zu kontrollieren, und neues Verbandsmaterial auf die Wunde aufgebracht werden muss. Zusätzlich wird der Heilungsprozess nicht durch das Entfernen gestört. Des Weiteren können dem Patienten durch einen Verbandswechsel möglicherweise entstehende Schmerzen verhindert werden.

Da die Änderung bereits von außen sichtbar ist, kann der Heilungsprozess auf einfache Art und Weise und mit geringem Aufwand überwacht werden.

Der Vorteil von Ausführungsbeispielen der Erfindung liegt insbesondere in der Selektivität der eingesetzten Indikatormaterialien gegenüber bestimmten Analyten. Dadurch ist es möglich, Analyte spezifisch zu erkennen, indem für jeden Analyten selektiv ein damit reagierender Rezeptor bzw. eine damit reagierende Rezeptorgruppe ausgewählt bzw. vorgesehen wird. Die Auswahl eines Rezeptors, beispielsweise eines Chromophors zur Erfassung von Aminen, kann dabei je nach Anwendung erfolgen.

Beispielsweise kann zur Erkennung von Aminen und/oder Alkoholen ein Chromophorensystem genutzt werden, das mit einer oder mehreren Trifluoracetylgruppen ausgestattet ist. Die Trifluoracetylgruppe erkennt selektiv Amine und/oder Alkohole, wobei die Reaktion der Rezeptorgruppe mit dem Amin bzw. den Aminen eine Änderung der optischen Eigenschaften des Chromophors hervorruft, die detektiert (z.B. visuell wahrgenommen oder spektroskopiert) werden kann.

Abhängig davon, welcher Analyt erfasst werden soll, wird bei Ausführungsbeispielen ein passender Rezeptor bzw. eine passende Rezeptorgruppe verwendet.

Bei Ausführungsbeispielen der Erfindung kann das Kontrollelement einen Rezeptor bzw. eine Rezeptorgruppe aufweisen, die aus der folgenden Gruppe ausgewählt ist:
- Trifluoracetylgruppen als selektive Rezeptorgruppen für Amine sowie Alkohole;
- Tricyanovinylgruppen als selektive Rezeptorgruppen für primäre und sekundäre Amine;
- Pyrylium-Funktionen als selektive Rezeptorgruppen für primäre Amine;
- Maleimidfunktionen als selektive Rezeptorgruppen für Thiole;
- Hydrazinfunktionen als selektive Rezeptorgruppen für Aldehyde und Ketone;
- Aminogruppen als selektive Rezeptorgruppen für Aldehyde und Ketone;
- Boronsäurefunktionen als selektive Rezeptorgruppen für Diole;
- Fluorescein-sulfonatgruppen als selektive Rezeptorgruppen für Wasserstoffperoxid;
- 1,2-Diaminobenzolgruppen als selektive Rezeptorgruppen für Stickoxide;
- Aldehydfunktionen als selektive Rezeptorgruppen für Aminosäuren; und
- Redoxsysteme als selektive Rezeptorgruppen für reaktive Sauerstoffspezies.

Als Redoxsysteme können bei Ausführungsbeispielen der Erfindung Metall-Ligand-Komplexe verwendet werden, wobei eine Erkennungsreaktion dann am Metallzentrum stattfinden kann.

Andere Rezeptoren, die selektiv eine sichtbare Änderung der Kontrollfläche für die angegebenen Analyten hervorrufen können, die über die hierin genannten Rezeptoren hinausgehen, sind Fachleuten bekannt und bedürfen hierin keiner weiteren Erläuterung. Erfindungsgemäß verwendete Rezeptoren sind selektiv für den oder die jeweiligen Analyten und sprechen nicht oder nur in geringem, für die Erfassung nicht relevantem Umfang auf andere Parameter, wie z.B. Änderungen des pH-Werts, an. Die Reaktion bekannter pH-Wert-Indikatoren ist unspezifisch, d.h. nicht selektiv, und lässt keine genaue Aussage über den Analyten zu.

Bei Ausführungsbeispielen der Erfindung können durch die entsprechenden Rezeptoren Analyte, wie z.B. Amine, direkt und selektiv erkannt werden. Im Gegensatz dazu ist beispielsweise eine Farbänderung durch Metalloprotease ein indirekter Nachweis, da die Protease die Gelatine zersetzt, wodurch die Freigabe des Farbstoffs ausgelöst wird.

Bei der vorliegenden Erfindung wird eine ionenundurchlässige Matrix verwendet, die zwischen der Wunde und dem Kontrollelement angeordnet ist, wenn das Verbandsmaterial auf die Wunde aufgebracht ist. Die ionenundurchlässige Matrix verhindert, dass Ionen mit dem Kontrollelement in Berührung kommen, so dass eine Erkennung von Ionen verhindert wird. Somit kann eine pH-Reaktion und somit ein Ansprechen auf pH-Wert-Änderungen verhindert werden.

Bei Ausführungsbeispielen der Erfindung kann die Selektivität durch Einbettung des oder der Rezeptoren, beispielsweise Farbstoffe, in poröse Strukturen (beispielsweise Mikro- oder Nanopartikel, oder poröse Metalloxidfilme), beeinflusst werden, so dass nur Analyte bestimmter Größe und Struktur in die pörosen Strukturen eindringen und mit den Rezeptoren, beispielsweise Farbstoffen, reagieren können.

Ausführungsbeispiele gemäß der Erfindung werden nachfolgend Bezug nehmend auf die beiliegenden Figuren näher erläutert. Es zeigen:
- Fig. 1A: eine schematische Darstellung einer Draufsicht auf ein Verbandsmaterial zur Überwachung einer Wundheilung;
- Fig. 1B, 1C, 1D: eine schematische Darstellung eines Querschnitts eines Verbandsmaterials zur Überwachung einer Wundheilung;
- Fig. 2: eine schematische Darstellung eines Querschnitts eines Verbandsmaterials zur Überwachung einer Wundheilung und einer Zwischenschicht;
- Fig. 3: eine schematische Darstellung eines Querschnitts eines Verbandsmaterials zur Überwachung einer Wundheilung mit einer Trägerschicht;
- Fig. 4A,4B: eine schematische Darstellung einer Draufsicht eines Verbandsmaterials zur Überwachung einer Wundheilung mit und ohne einer sichtbaren Änderung eines Teils der Gesamtfläche der Kontrollfläche;
- Fig. 5A, 5B: eine schematische Darstellung einer Draufsicht eines Pflasters mit Kontrollpunkten in unterschiedlichen Zuständen; und
- Fig. 6: ein Flussdiagramm eines Verfahrens zum Erkennen einer Änderung einer Eigenschaft einer Wunde.

Im Folgenden werden teilweise für Objekte und Funktionseinheiten, die gleiche oder ähnliche funktionelle Eigenschaften aufweisen, gleiche Bezugszeichen verwendet. Des Weiteren können optionale Merkmale der verschiedenen Ausführungsbeispiele miteinander kombinierbar oder zueinander austauschbar sein.

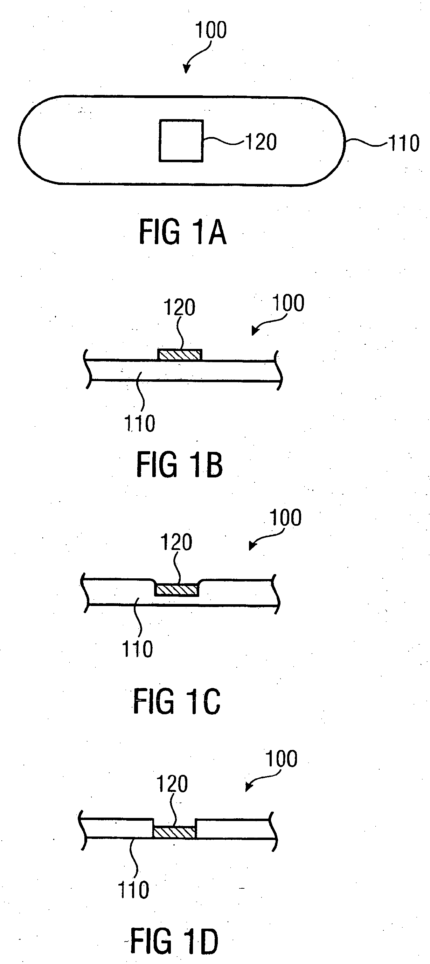

Fig. 1A bis 1D zeigen schematische Darstellungen eines Verbandsmaterials 100 zur Überwachung einer Wundheilung als Ausführungsbeispiele gemäß der Erfindung. Das Verbandsmaterial 100 umfasst ein Verbandsgrundmaterial 110 und ein Kontrollelement 120. Das Kontrollelement 120 ist in oder auf dem Verbandsgrundmaterial 110 angeordnet, so dass eine Kontrollfläche des Kontrollelements 120 sichtbar ist und eine flüssige oder gasförmige Absonderung einer Wunde mit dem Kontrollelement 120 in Kontakt ist, wenn das Verbandsmaterial 100 auf die Wunde aufgebracht ist. Zusätzlich ruft das Kontrollelement 120 bei einer Änderung einer Eigenschaft der Wunde, basierend auf einer chemischen Reaktion eine sichtbare Änderung der Kontrollfläche hervor, um die Überwachung der Wundheilung zu ermöglichen.

Durch die beschriebene Anordnung des Kontrollelements 120 kann gewährleistet werden, dass eine Änderung der Kontrollfläche, verursacht durch eine Änderung einer Eigenschaft der Wunde, sichtbar ist, obwohl sich das Verbandsmaterial 100 noch auf der Wunde befindet. In anderen Worten, das Kontrollelement 120 ist in oder auf dem Verbandsgrundmaterial 110 so angeordnet, dass die Wundheilung überwachbar ist, ohne das Verbandsmaterial 100 von der Wunde zu entfernen. Dadurch kann auf eine einfache Art und Weise und mit geringem Aufwand die Wundheilung sowohl durch medizinisches als auch durch nichtmedizinisches Personal sowie dem Patienten selbst verfolgt werden. Zusätzlich kann einerseits das möglicherweise schmerzhafte Entfernen des Verbandsmaterials 100 zur Kontrolle der Wundheilung vermieden werden, wodurch andererseits auch Kosten für neues Verbandsmaterial gespart werden können. Durch das beschriebene Konzept kann die Wundheilung beispielsweise ohne Verbandswechsel und ohne Verwendung diagnostischer Blattmaterialien überwacht werden.

Zusätzlich kann das Kontrollelement 120 ausgelegt sein, eine Stärke der sichtbaren Änderung der Kontrollfläche proportional zu einer Stärke der Änderung der Eigenschaft der Wunde hervorzurufen. Beispielsweise kann sich die Kontrollfläche umso stärker verfärben, je größer die Änderung der Eigenschaft der Wunde ist. Dadurch kann ein Anhaltspunkt für das Ausmaß einer Störung einer Wundheilung ermöglicht werden.

Die flüssige oder gasförmige Absonderung kann beispielsweise eine Wundflüssigkeit der Wunde, eine gasförmige Absonderung von Aminen in der Wunde oder der Wundflüssigkeit der Wunde oder eine andere Absonderung einer feuchten oder trockenen Wunde sein.

Das Verbandsmaterial 100 kann beispielsweise ein Pflaster oder ein Verband sein. Bei dem Verbandsgrundmaterial 110 kann es sich um jegliches bekanntes Material, das beispielsweise zur Herstellung von Pflaster oder von Verbänden verwendet wird, handeln. Dabei kann das Verbandsgrundmaterial 110 aus einem oder mehreren verschiedenen Materialien bestehen.

Das Kontrollelement 120 ist beispielsweise ein Träger einer Substanz, die auf einer Änderung einer Eigenschaft einer Wunde, basierend auf einer chemischen Reaktion z.B. mit einer Änderung seiner optischen oder chemischen Eigenschaften reagiert. Beispielsweise kann die Substanz, verursacht durch die Änderung der Eigenschaft der Wunde, ihre Farbe, ihre Helligkeit, ihre Fluoreszenz, ihre Lumineszenz oder ihre chemische Reaktivität mit anderen Stoffen ändern. Dies kann direkte sichtbare Änderungen der Kontrollfläche hervorrufen, wenn die Substanz beispielsweise direkt auf die Kontrollfläche aufgebracht ist, oder indirekt eine sichtbare Änderung der Kontrollfläche bewirken. Die Kontrollfläche des Kontrollelements 120 kann auf einer der Wunde abgewandten Seite des Kontrollelements 120 oder auf einer der Wunde zugewandten Seite des Kontrollelements 120 angeordnet sein. Ist die Kontrollfläche auf der der Wunde zugewandten Seite des Kontrollelements 120 angeordnet, so kann das Kontrollelement zumindest teilweise durchsichtig oder zumindest zu einem ausreichenden Grade transparent sein, so dass die Kontrollfläche sichtbar ist, wenn das Verbandsmaterial auf die Wunde aufgebracht ist. Zum Beispiel kann das Kontrollelement 120 ein biokompatibles Polymer oder ein Zellstoff sein, an dem eine chemisch reaktive Substanz gebunden ist.

Beispielsweise kann das Kontrollelement 120 ein biokompatibles Polymer mit einem kovalent gebundenen Rezeptor sein, der für den gewünschten Analyt sensitiv ist. Der Rezeptor kann beispielsweise ein entsprechend sensitiver Farbstoff sein. Der sensitive Farbstoff kann dann beispielsweise auf der Kontrollfläche des Kontrollelements 120 gebunden sein. Zusätzlich kann das biokompatible Polymer durchsichtig sein, wenn die Kontrollfläche auf der der Wunde zugewandten Seite des Polymers angeordnet ist. Beispielsweise kann die Substanz des Kontrollwerts bei einer ersten Konzentration des Analyts, die durch flüssige oder gasförmige Absonderungen der Wunde verursacht wird, eine erste Farbe haben und bei einer zweiten Konzentration des Analyts, die durch flüssige oder gasförmige Absonderung der Wunde verursacht wird, eine zweite Farbe aufweisen.

Deshalb ist eine Kontrolle der Wunde von außen, das heißt, ohne Entfernen des Wundverbandes (Verbandsmaterials) von großem Wert im Bereich der Patientenversorgung.

Das Kontrollelement 120 kann des Weiteren aus einem biegsamen Material bestehen, um sich einer Form einer Oberfläche, auf die das Verbandsmaterial 100 aufgebracht wird, anzupassen. Dadurch kann sich dann sowohl das Verbandsgrundmaterial 110 als auch das Kontrollelement an die Form des Körperteils und der Wunde anpassen, an dem das Verbandsmaterial 100 verwendet wird.

Das Kontrollelement 120 kann beispielsweise auch als Kontrollstreifen oder Kontrollpunkt bezeichnet werden.

Eine Eigenschaft einer Wunde kann jeglicher Parameter sein, bei dem sich eine Änderung des gewünschten Analyts, basierend auf einer chemischen Reaktion feststellen lässt. Beispielsweise kann es sich dabei um eine Konzentration von reaktiven Sauerstoffspezies, Aminen, Alkoholen, Diolen, Carbonsäure, Aldehyden, Ketonen, Aminosäuren, Wasserstoffperoxid, Stickoxid, Thiolen, Mercaptanen, Carbonsäuren und/oder Schwefelwasserstoff handeln. Zum Beispiel kann eine Änderung einer Amin-Konzentration, die der durch flüssige oder gasförmige Absonderungen der Wunde verursacht wird, durch ein Amin-sensitives Kontrollelement 120 sichtbar gemacht werden. Dazu kann sich beispielsweise auf der Kontrollfläche des Kontrollelements 120 ein Amin-sensitiver Farbstoff befinden, der bei Änderung der Amin-Konzentration der flüssigen oder gasförmigen Absonderung eine sichtbare Farbänderung erfährt. Zum Beispiel können entsprechende Änderungen durch Reaktionen mit chemilumineszenten, chromogenen und/oder fluorogenen Substraten angezeigt werden.

Die Änderung der Eigenschaft der Wunde kann beispielsweise einer Änderung einer Eigenschaft einer Wundflüssigkeit der Wunde entsprechen.

Fig. 1A zeigt ein Beispiel einer schematischen Darstellung einer Draufsicht auf ein Verbandsmaterial 100 zur Überwachung einer Wundheilung. Dabei kann das Verbandsgrundmaterial eine beliebige Form, wie beispielsweise Formen von herkömmlichen Pflastern oder Verbänden oder andere Formen, aufweisen. Das Kontrollelement 120 ist in oder auf dem Verbandsgrundmaterial 110 angeordnet. Dabei ist wie gezeigt, zumindest eine Kontrollfläche des Kontrollelements 120 sichtbar.

Fig. 1B bis 1D zeigen Beispiele von Querschnitten von Verbandsmaterialien 100. Dabei kann das Kontrollelement 120, wie in Fig. 1B gezeigt, auf dem Verbandsgrundmaterial 110 oder, wie in Fig. 1C gezeigt, in dem Verbandsgrundmaterial 110 angeordnet sein, so dass zumindest ein Teil des Verbandsgrundmaterials 110 (oder das Verbandsgrundmaterial mit einem Teil seiner Dicke) zwischen dem Kontrollelement 120 und der Wunde angeordnet ist, wenn das Verbandsmaterial 100 auf die Wunde aufgebracht ist.

Alternativ kann das Verbandsgrundmaterial 110, wie in Fig. 1D gezeigt, an der Stelle des Kontrollelements 120 unterbrochen sein. Das Kontrollelement 120 kann dann in direktem Kontakt mit der Wunde stehen.

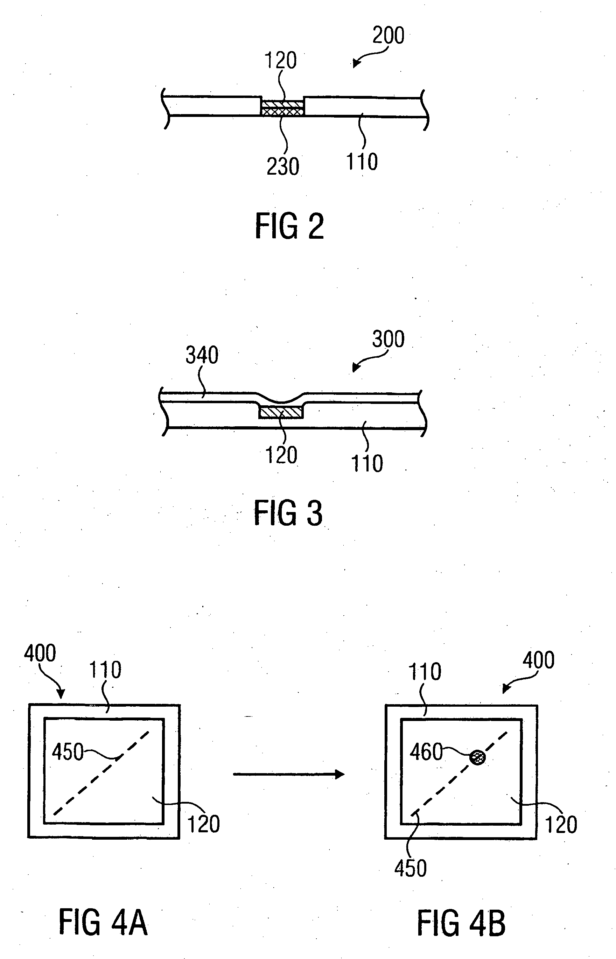

Fig. 2 zeigt eine schematische Darstellung eines Querschnitts eines Verbandsmaterials 200 zur Überwachung einer Wundheilung entsprechend eines Ausführungsbeispiels gemäß der Erfindung. Das Verbandsmaterial 200 ist ähnlich dem in Fig. 1D gezeigten Verbandsmaterial, umfasst jedoch zusätzlich eine Zwischenschicht 230. Die Zwischenschicht 230 ist zwischen dem Kontrollelement 120 und der Wunde angeordnet, wenn das Verbandsmaterial 200 auf die Wunde aufgebracht ist. Die Zwischenschicht 230 kann beispielsweise ein Material aufweisen, das einen guten Transport von flüssigen oder gasförmigen Absonderung von der Wunde zu dem Kontrollelement 120 ermöglicht. Dies kann beispielsweise aufgrund einer Kapillarwirkung eines Zellstoffs passieren.

Die Zwischenschicht 230 weist eine ionenundurchlässige Matrix auf, die somit zwischen der Wunde und dem Kontrollelement angeordnet ist, wenn das Verbandsmaterial auf die Wunde aufgebracht ist. Dadurch kann verhindert werden, dass Ionen mit dem Kontrollelement in Berührung kommen, so dass eine pH-Reaktion und somit ein Ansprechen auf pH-Wert-Änderungen verhindert werden kann.

Fig. 3 zeigt eine weitere schematische Darstellung eines Querschnitts eines Verbandsmaterials 300 zur Überwachung einer Wundheilung entsprechend eines Ausführungsbeispiels gemäß der Erfindung. Das Verbandsmaterial 300 ähnelt dem in Fig. 1C gezeigten Aufbau, umfasst jedoch zusätzlich eine Trägerschicht 340. Die Trägerschicht 340 ist auf der auf der Wunde abgewandten Seite des Verbandsgrundmaterials 110 und des Kontrollelements 120 angeordnet, und dient beispielsweise zur Fixierung der Position des Kontrollelements 120 in oder auf dem Verbandsgrundmaterial 110. Die Trägerschicht 340 ist zumindest in einem Bereich des Kontrollelements 120 durchsichtig oder zumindest zu einem Grad transparent, so dass die Kontrollfläche des Kontrollelements 120 sichtbar ist. Die Trägerschicht 340 kann optional über das Verbandsgrundmaterial 110 hinausragen und in dem über das Verbandsgrundmaterial 110 hinausragenden Teil auf der der Wunde zugewandten Seite ein Haftmittel aufweisen, um das Verbandsmaterial 300 auf dem Körperteil mit der Wunde festzukleben.

Alternativ zu dem in Fig. 3 gezeigten Ausbau, kann das Verbandsgrundmaterial 110 entsprechend den in Fig. 1D gezeigten Schema im Bereich des Kontrollelements 120 nicht vorhanden sein oder es kann durch eine Zwischenschicht, wie in Fig. 2D gezeigt, ersetzt werden. Genauso kann das Kontrollelement 120 auf dem Verbandsgrundmaterial 110 angeordnet sein, wie in Fig. 1B dargestellt.

Fig. 4A und 4B zeigen eine schematische Darstellung einer Draufsicht eines Verbandsmaterials 400 zur Überwachung einer Wundheilung entsprechend eines Ausführungsbeispiels gemäß der Erfindung. Das Verbandsmaterial 400 umfasst ein großflächiges Kontrollelement 120, durch das eine Überwachung von größeren Wunden ermöglicht wird. Es kann zusätzlich zur Beurteilung, ob eine Änderung einer Eigenschaft der Wunde stattgefunden hat, auch eine Aussage über den Ort der Änderung und/oder das Ausmaß der Änderung ermöglichen.

Als Beispiel zeigt Fig. 4A eine große Wunde 450, über die sich das Kontrollelement 120 erstreckt. Tritt nun nur in einem Teil der Wunde eine Änderung einer Eigenschaft der Wunde auf, ist neben der Information, ob eine Änderung auftritt, auf eine Aussage über den Ort der Änderung und/oder das Ausmaß der Änderung wichtig. Dazu kann das Kontrollelement 120 ausgelegt sein, dass die sichtbare Änderung einen Teil der Gesamtfläche der Kontrollfläche betrifft, wobei eine Größe des betroffenen Teils der Gesamtfläche proportional zu einer Fläche der Wunde ist, in der sich die Eigenschaften der Wunde ändert oder ein Ort des betroffenen Teils der Gesamtfläche auf einem Ort der Wunde basiert, indem sich die Eigenschaft der Wunde ändert.

Ein Beispiel dafür ist in Fig. 4B gezeigt. Die sichtbare Änderung 460 der Kontrollfläche beschränkt sich auf einen Teil der Kontrollfläche. Der Ort der sichtbaren Änderung kann mit dem Ort der Wunde korreliert werden, in dem sich die Eigenschaft der Wunde ändert, und die Größe der sichtbaren Änderung kann proportional zu der Fläche der Wunde sein, in der sich die Eigenschaft der Wunde ändert. Dadurch kann eine Aussage über den Ort und/oder das räumliche Ausmaß einer Änderung der Eigenschaften der Wunde gemacht werden.

Um eine Wunde zu kontrollieren, müsste ein herkömmlicher Verband entfernt und eine Probe entnommen werden, was zeitaufwendig und für den Patienten unter Umständen schmerzhaft ist.

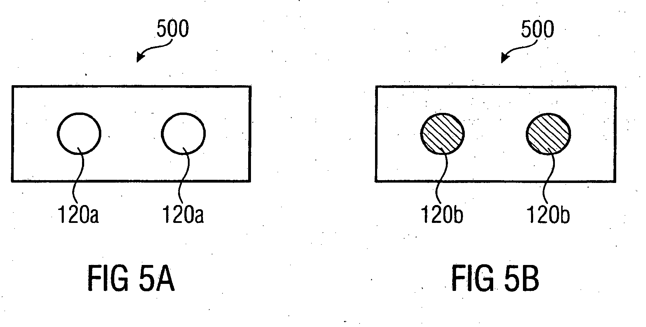

Deshalb wird zum Beispiel entsprechend des beschriebenen Konzepts die Herstellung eines "intelligenten Verbandsmaterials" vorgeschlagen, welches entsprechende Änderungen der Wunde durch Farbänderung eines Kontrollstreifens anzeigen kann. Dazu kann beispielsweise ein entsprechend sensitiver Farbstoff (Rezeptor) kovalent an ein biokompatibles Polymer gebunden werden und dieses Polymer als Kontrollstreifen in das Pflaster, bzw. den Wundverband (das Verbandsmaterial) integriert (Fig. 5a und 5b) werden.

Der verwendete sensitive Farbstoff kann beispielsweise den Farbumschlag in einem für den Heilverlauf einer Wunde kritischen Bereich haben und kann eine deutliche Farbänderung zeigen. In Fig. 5a ist ein Pflaster 500 mit integrierten Farbstoff-Polymer-Kontrollpunkten 120A bei einer ersten Konzentration eines Analyts dargestellt. Hier ist die Farbe der Kontrollpunkte z.B. orange (λ_{max, abs} = 450 nm). Beim Übergang über eine bestimmte Konzentration des Analyts erfolgt ein Farbumschlag nach z.B. blau (λ_{max, abs} = 600 nm). In Fig. 5b ist die deutliche Veränderung der Farbe der Kontrollpunkte 120B des Pflasters 500 sichtbar.

Die Entstehung von Analyten, insbesondere von reaktiven Sauerstoffspezies, Alkoholen, Diolen, Carbonsäure, Aldehyden, Ketonen, Aminosäuren, Wasserstoffperoxid, Stickoxid, Aminen, Thiolen, Schwefelwasserstoff, Carbonsäuren und Mercaptanen, kann auf Komplikationen bei der Heilung hinweisen. Ihre Entstehung kann also durch den Kontrollstreifen angezeigt werden und zusätzliche Hinweise auf Veränderungen der Wunde liefern. Analyte, wie z.B. Amine, können in trockenen und feuchten Wunden entstehen. Der anzunehmende Ablauf der Wundheilung einer feuchten oder trockenen Wunde ist prinzipiell identisch, die Trockenheit kann deren Ablauf erheblich verzögern.

Die Verwendung eines biokompatiblen Polymers und die kovalente Immobilisierung des Rezeptors, beispielsweise des sensitiven Farbstoffs, bei der Herstellung des Kontrollstreifens kann die Kontamination der Wunde mit schädlichen Substanzen verhindern.

Ein Vorteil eines Verbandsmaterials mit Farbkontrollstreifen liegt in der einfachen Handhabung. Eine Verwendung solch eines Kontrollstreifens im Verbandsmaterial könnte große Vorteile bei der Patientenbetreuung im Pflegedienstbereich, d. h. in Pflegeheimen und bei der häuslichen Patientenversorgung mit sich bringen. Das beschriebene Konzept kann die Patientenbetreuung für das nichtmedizinische Personal vereinfachen. Besonders die Pflegedienste im Bereich der Altenpflege sind überlastet. Neben der hygienischen Versorgung der Patienten, ihrer Verpflegung und sozialer Betreuung muss das Pflegepersonal mögliche Erkrankungen frühzeitig erkennen.

Ein häufig auftretendes Problem der Altenpflege ist die Bettlägerigkeit, die in vielen Fällen zum Wundliegen (Decubitus) der Patienten führt. Diese Erkrankung zeigt sich z.B. durch das Auftreten schmerzhafter Geschwüre und Infektionen, die schwer zu behandeln sind, da die Hauptursache, das ständige Liegen nicht beseitigt werden kann. Eine medikamentöse Behandlung der oft geschwächten Patienten birgt unter Umständen hohe Gesundheitsrisiken. Ein Früherkennung des Decubitus wäre also von großem Wert für eine Verbesserung der Lebensumstände dieser Menschen.

Das erfindungsgemäße Verbandsmaterial kann beispielsweise auch für Patienten mit großflächigen Wunden (z. B. auch Brandopfer) eine Verbesserung mit sich bringen, da gerade dort die Wundkontrolle besonders zeitaufwendig und schmerzhaft ist und ein häufiges Öffnen des Wundverbandes ein großes Infektionsrisiko birgt.

Wird der Kontrollstreifen in Pflastern für den häuslichen Gebrauch eingesetzt, kann er z. B. die Wundkontrolle bei Kindern vereinfachen. Eltern haben so die Möglichkeit, ihren Kindern die Entfernung des Pflasters zu ersparen, da auch hier z.B. die visuelle Überprüfung der Farbe des Kontrollstreifens genügt.

Für den Patienten ergeben sich also mehrere Vorteile. Man hat die Möglichkeit Komplikationen bei der Wundbeheilung ohne schmerzhaftes Entfernen der Verbände zu erkennen. Das führt zu einer Zeitersparnis bei der Wundenkontrolle, da statt der Entfernung des Verbandes und einer Probenentnahme die visuelle Überprüfung der Farbe des Kontrollstreifens genügt.

Mit der Farbänderung wird zudem eine leicht zu deutende Information erzeugt, die es sogar dem Laien (und dem Patienten) ermöglicht, festzustellen, dass in der Wunde eine Veränderung stattgefunden hat. Dieser Umstand trägt zur Früherkennung von Komplikationen bei.

Alternativ zu den oben beschriebenen Indikatoren, Polymeren und Verbandsgrundmaterialien können auch andere Indikatoren, andere Polymere zur Immobilisierung oder ein anderes Verbandsmaterial verwenden.

Entsprechende Rezeptoren zur Detektion der angegeben Analyte, beispielsweise in Form von Indikatorfarbstoffen sind Fachleuten bekannt.

Lediglich beispielhaft sei auf die folgenden Ausführungen verwiesen. Erhöhte Mengen an reaktiven Oxidations-Spezies sind für einen verzögerten Wundheilungsprozess verantwortlich. Zur Bestimmung von reaktiven Sauerstoffspezies können beispielsweise an Wundverbände immobilisierte Redoxindikatoren wie z.B. Methylenblau und Indigo verwendet werden. Diese Redoxindikatoren zeigen Farbänderungen mit Verbindungen wie Wasserstoffperoxid, Singulett-Sauerstoff, Superoxidanionen und deren Radikale, Hydroxylradikale, Stickstoffmonoxid und Peroxynitrit (A. Lobnik, M. Cajlakovic, Sol-gel based optical sensor for continuous determination of dissolved hydrogen peroxide, Sensors and Actuators B Chemical, 74 (2001) 194-199; G. J. Mohr, New chromogenic and fluorogenic reagents and sensors for neutral and ionic analytes based on covalent bond formation - A review of recent developments, Analytical and Bioanalytical Chemistry, 386 (2006) 1201-1214).

Über immobilisierte Indikatorfarbstoffe für Aldehyde und Ketone (G. J. Mohr, U. E. Spichiger, W. Jona, H. Langhals, Using N-aminoperylene-3,4:9,10-tetracarboxyl-bisimide as a fluorogenic reactand in the optical sensing of aqueous propionaldehyde, Analytical Chemistry, 72 (2000) 1084-1087) können Intermediate der Oxidation detektiert werden, wie z.B. Malondialdehyd, Isoprostan oder 4-Hydroxynonenal. Des Weiteren können Antioxidantien, wie Ascorbinsäure und Harnsäure über an Wundverbände immobilisierte Indikatorfarbstoffe für Carbonsäuren detektiert warden (A. B. Descalzo, K. Rurack, H. Weisshoff, R. Martinez-Manez, M. D. Marcos, P. Amoros, K. Hoffmann, J. Soto, Rational design of a chromo- and fluorogenic hybrid chemosensor material for the detection of long-chain carboxylates, Journal of the American Chemical Society 127 (2005) 184-200).

Erhöhte Mengen an Proteasen in der Wunde sind für einen verzögerten Wundheilungsprozess verantwortlich. Erhöhte Mengen an Bakterien in der Wunde erzeugen potentiell toxische Reaktionsprodukte. Zur Bestimmung von Abbauprodukten von, beispielsweise, Aminosäuren und Peptiden können spezielle Indikatoren für biogene Amine (G. J. Mohr, C. Demuth, U. E. Spichiger, Application of chromogenic and fluorogenic reactands in the optical sensing of dissolved aliphatic amines, Analytical Chemistry, 70 (1998) 3868-3873; G. J. Mohr, N. Tirelli, C. Lohse, U. E. Spichiger, Development of chromogenic copolymers for optical detection of amines, Advanced Materials, 10 (1998) 1353-1357; G. J. Mohr, A tricyanovinyl azobenzene dye used for the optical detection of amines via a chemical reaction in polymer layers, Dyes and Pigments, 62 (2004) 77-81; B. K. Hoefelschweiger, A. Duerkop, O. S. Wolfbeis, Novel type of general protein assay using a chromogenic and fluorogenic amine-reactive probe, Analytical Biochemistry 344 (2005) 122-129) eingesetzt warden, wie z.B. an Wundverbände immobilisierte Tricyanovinyl-, Trifluoracetyl- oder Pyryliumfarbstoffe. Es können auch selektive Indikatoren für Aminosäuren, Schwefelwaserstoff und Mercaptane eingesetzt warden (G. J. Mohr, Covalent bond formation as an analytical tool to optically detect neutral and anionic analytes, Sensors and Actuators B, 107 (2005) 2-13.).

Einige Ausführungsbeispiele gemäß der Erfindung beziehen sich auf eine Verwendung des beschriebenen Verbandsmaterials für stationäre, ambulante und/oder häusliche Wundversorgung sowie als Cluster für den häuslichen Gebrauch.

Durch das beschriebene Konzept kann beispielsweise ein intelligentes Pflaster zur optischen Wundüberwachung bereitgestellt werden. Angestrebt ist eine Vereinfachung der Wundenkontrolle durch Einbringen eines Kontrollstreifens (Kontrollelement) in das Verbandsmaterial. Der Kontrollstreifen soll z.B. durch einen einfachen Farbumschlag Veränderungen in der Wunde anzeigen, somit dem Pflegepersonal Zeit und dem Patienten das schmerzhafte Entfernen der Verbände ersparen, wenn dies noch nicht erforderlich ist. Eine Farbänderung eines Kontrollstreifens im Verband kann auch von nichtmedizinischem Personal und auch vom Patienten leicht erkannt werden und trägt damit zur Früherkennung von Komplikationen bei der Wundheilung bei.

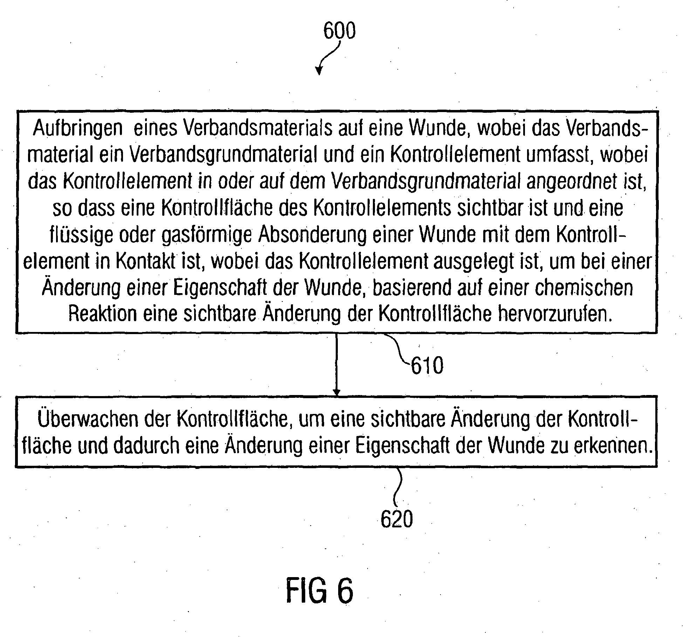

Fig. 6 zeigt ein Flussdiagramm eines Verfahrens 600 zum Erkennen einer Änderung einer Eigenschaft einer Wunde entsprechend eines Ausführungsbeispiels gemäß der Erfindung. Das Verfahren 600 umfasst ein Aufbringen 610 eines Verbandsmaterials auf eine Wunde, wobei das Verbandsmaterial ein Verbandsgrundmaterial und ein Kontrollelement aufweist. Das Kontrollelement ist in oder auf dem Verbandsgrundmaterial angeordnet, so dass eine Kontrollfläche des Kontrollelements sichtbar ist und eine flüssige oder gasförmige Absonderung der Wunde mit dem Kontrollelement in Kontakt ist. Zusätzlich ist das Kontrollelement ausgelegt, um bei einer Änderung einer Eigenschaft der Wunde basierend auf einer chemischen Reaktion eine sichtbare Änderung der Kontrollfläche hervorzurufen, um die Überwachung der Wundheilung zu ermöglichen. Des Weiteren umfasst das Verfahren 600 ein Überwachen 620 der Kontrollfläche, um eine sichtbare Änderung der Kontrollfläche und dadurch eine Änderung einer Eigenschaft der Wunde zu erkennen.

Durch das Verfahren 600 (diagnostisches Verfahren) kann sozusagen ein Messwert (eine Änderung einer Eigenschaft einer Wunde) erfasst werden. Basierend auf diesem Messwert kann z.B. medizinisches Personal weitere Schritte einleiten (z.B. Verband wechseln, Wunde behandeln).

Das Verfahren 600 kann optional um weitere Schritte, die im Zusammenhang mit dem Verbandsmaterial beschrieben wurden, erweitert werden.

Einige Ausführungsbeispiele gemäß der Erfindung beziehen sich auf ein Verfahren zum Herstellen eines Verbandsmaterials. Das Verfahren umfasst ein Anordnen eines Koritrollelements in oder auf einem Verbandsgrundmaterial, so dass eine Kontrollfläche des Kontrollelements sichtbar ist und eine flüssige oder gasförmige Absonderung einer Wunde mit dem Kontrollelement in Kontakt ist, wenn das Verbandsmaterial auf die Wunde aufgebracht ist. Dabei ist das Kontrollelement ausgelegt, um bei einer Änderung einer Eigenschaft der Wunde, basierend auf einer chemischen Reaktion eine sichtbare Änderung der Kontrollfläche hervorzurufen, um die Überwachung der Wundheilung zu ermöglichen.

Obwohl manche Aspekte im Zusammenhang mit einer Vorrichtung beschrieben wurden, versteht es sich, dass diese Aspekte auch eine Beschreibung des entsprechenden Verfahrens darstellen, sodass ein Block oder ein Bauelement einer Vorrichtung auch als ein entsprechender Verfahrensschritt oder als ein Merkmal eines Verfahrensschrittes zu verstehen ist. Analog dazu stellen Aspekte, die im Zusammenhang mit einem oder als ein Verfahrensschritt beschrieben wurden, auch eine Beschreibung eines entsprechenden Blocks oder Details oder Merkmals einer entsprechenden Vorrichtung dar.

## Patentansprüche

1. Verbandsmaterial (100, 200, 300, 400, 500) zur Überwachung einer Wundheilung, mit folgenden Merkmalen:
einem Verbandsgrundmaterial (110);
einem Kontrollelement (120), das in oder auf dem Verbandsgrundmaterial (110) angeordnet ist, so dass eine Kontrollfläche des Kontrollelements (120) sichtbar ist und eine flüssige oder gasförmige Absonderung einer Wunde mit dem Kontrollelement in Kontakt ist, wenn das Verbandsmaterial auf die Wunde aufgebracht ist; und
einer ionenundurchlässigen Matrix, die zwischen der Wunde und dem Kontrollelement angeordnet ist, wenn das Verbandsmaterial auf die Wunde aufgebracht ist,
wobei das Kontrollelement (120) ausgelegt ist, um bei einer Änderung einer Eigenschaft der Wunde, basierend auf einer durch die flüssige oder gasförmige Absonderung bedingte chemischen Reaktion eine sichtbare Änderung der Kontrollfläche hervorzurufen, um die Überwachung der Wundheilung zu ermöglichen,
wobei das Kontrollelement zumindest einen Rezeptor aufweist, der ausgelegt ist, um selektiv auf einen oder mehrere Analyten anzusprechen, der oder die aus der Gruppe ausgewählt sind, die reaktive Sauerstoffspezies, Amine, Alkohole, Thiole, Aldehyde, Ketone, Diole, Wasserstoffperoxid, Stickoxid, Aminosäuren, Carbonsäuren, Schwefelwasserstoff und Mercaptane enthält.

2. Verbandsmaterial gemäß Anspruch 1, wobei das Kontrollelement (120) in oder auf dem Verbandsgrundmaterial (110) angeordnet ist, so dass die Wundheilung überwachbar ist, ohne das Verbandsmaterial von der Wunde zu entfernen.

3. Verbandsmaterial gemäß Anspruch 1 oder 2, wobei das Kontrollelement (120) ausgelegt ist, um bei einer Änderung der Eigenschaft der Wunde eine Farbe der Kontrollfläche zu ändern.

4. Verbandsmaterial gemäß einem der Ansprüche 1 bis 3, wobei die Kontrollfläche des Kontrollelements (120) eine der Wunde zugewandte Seite des Kontrollelements (120) ist, und wobei das Kontrollelement (120) durchsichtig ist, so dass die Kontrollfläche sichtbar ist, wenn das Verbandsmaterial auf die Wunde aufgebracht ist.

5. Verbandsmaterial gemäß einem der Ansprüche 1 bis 4, bei dem das Kontrollelement Indikatorfarbstoffe zur Detektion von reaktiven Sauerstoffspezies, Alkoholen, Diolen, Carbonsäure, Aldehyden, Ketonen, Aminosäuren, Wasserstoffperoxid, Stickoxid, Aminen, Thiolen, Mercaptanen, Carbonsäuren und/oder Schwefelwasserstoff aufweist.

6. Verbandsmaterial gemäß einem der Ansprüche 1 bis 5, wobei ein Teil des Verbandsgrundmaterials (110) zwischen dem Kontrollelement (120) und der Wunde angeordnet ist, wenn das Verbandsmaterial auf die Wunde aufgebracht ist.

7. Verbandsmaterial gemäß einem der Ansprüche 1 bis 5, das ferner eine Zwischenschicht (230) aufweist, die zwischen dem Kontrollelement (120) und der Wunde angeordnet ist, wobei die Zwischenschicht (230) ausgelegt ist, um die flüssige oder gasförmige Absonderung von der Wunde zu dem Kontrollelement (120) zu transportieren.

8. Verbandsmaterials gemäß einem der Ansprüche 1 bis 7, wobei das Kontrollelement (120) aus einem biegsamen Material besteht, um sich einer Form einer Oberfläche, auf die das Verbandsmaterial aufgebracht wird, anzupassen.

9. Verbandsmaterial gemäß einem der Ansprüche 1 bis 8, wobei die sichtbare Änderung einen Teil einer Gesamtfläche der Kontrollfläche betrifft, wobei eine Größe des betroffenen Teils der Gesamtfläche proportional zu einer Fläche der Wunde ist, in der sich die Eigenschaft der Wunde ändert, oder wobei ein Ort des betroffenen Teils der Gesamtfläche auf einem Ort der Wunde basiert, an dem sich die Eigenschaft der Wunde ändert.

10. Verbandsmaterial gemäß einem der Ansprüche 1 bis 9, wobei die flüssige oder gasförmige Absonderung eine Wundflüssigkeit der Wunde ist.

11. Verbandsmaterial gemäß einem der Ansprüche 1 bis 10, wobei die Änderung der Eigenschaft der Wunde einer Änderung einer Eigenschaft einer Wundflüssigkeit der Wunde entspricht.

12. Verbandsmaterial gemäß einem der Ansprüche 1 bis 11, bei dem der oder die Rezeptoren in poröse Strukturen eingebracht sind, wobei die porösen Strukturen angepasst sind, um nur Analyte bestimmter Größe und Struktur eindringen zu lassen.

13. Pflaster umfassend ein Verbandsmaterial gemäß einem der Ansprüche 1 bis 11.

14. Verfahren (600) zum Erkennen einer Änderung einer Eigenschaft einer Wunde, mit folgenden Schritten:
Aufbringen (610) eines Verbandsmaterials auf eine Wunde, wobei das Verbandsmaterial ein Verbandsgrundmaterial und ein Kontrollelement umfasst, wobei das Kontrollelement in oder auf dem Verbandsgrundmaterial angeordnet ist, so dass eine Kontrollfläche des Kontrollelements sichtbar ist und eine flüssige oder gasförmige Absonderung einer Wunde mit dem Kontrollelement in Kontakt ist, wobei eine ionenundurchlässige Matrix zwischen der Wunde und dem Kontrollelement angeordnet ist, wobei das Kontrollelement ausgelegt ist, um bei einer Änderung einer Eigenschaft der Wunde, basierend auf einer chemischen Reaktion eine sichtbare Änderung der Kontrollfläche hervorzurufen; und
Überwachen (620) der Kontrollfläche, um eine sichtbare Änderung der Kontrollfläche und dadurch eine Änderung einer Eigenschaft der Wunde zu erkennen,
wobei das Kontrollelement zumindest einen Rezeptor aufweist, der ausgelegt ist, um selektiv auf einen oder mehrere Analyten anzusprechen, der oder die aus der Gruppe ausgewählt sind, die reaktive Sauerstoffspezies, Amine, Alkohole, Thiole, Aldehyde, Ketone, Diole, Wasserstoffperoxid, Stickoxid, Aminosäuren, Carbonsäuren, Schwefelwasserstoff und Mercaptane enthält.

15. Verfahren zum Herstellen eines Verbandsmaterials, mit folgendem Schritt:
Anordnen eines Kontrollelements in oder auf einem Verbandsgrundmaterial, so dass eine Kontrollfläche des Kontrollelements sichtbar ist und eine flüssige oder gasförmige Absonderung einer Wunde mit dem Kontrollelement in Kontakt ist, wenn das Verbandsmaterial auf die Wunde aufgebracht ist, und so dass eine ionenundurchlässige Matrix zwischen der Wunde und dem Kontrollelement angeordnet ist, wenn das Verbandsmaterial auf die Wunde aufgebracht ist, wobei das Kontrollelement äusgelegt ist, um bei einer Änderung einer Eigenschaft der Wunde basierend auf einer chemischen Reaktion eine sichtbare Änderung der Kontrollfläche hervorzurufen, um eine Überwachung der Wundheilung zu ermöglichen, wobei das Kontrollelement zumindest einen Rezeptor aufweist, der ausgelegt ist, um selektiv auf einen oder mehrere Analyten anzusprechen, der oder die aus der Gruppe ausgewählt sind, die reaktive Sauerstoffspezies, Amine, Alkohole, Thiole, Aldehyde, Ketone, Diole, Wasserstoffperoxid, Stickoxid, Aminosäuren, Carbonsäuren, Schwefelwasserstoff und Mercaptane enthält.

## Claims

1. Dressing material (100, 200, 300, 400, 500) for monitoring healing of a wound, comprising:
a basic dressing material (110);
a control element (120) disposed in or on the basic dressing material (110) so that a control area of the control element (120) is visible and that a liquid or gaseous secretion of a wound is in contact with the control element once the dressing material has been applied to the wound; and
an ion-impermeable matrix disposed between the wound and the control element once the dressing material has been applied to the wound,
the control element (120) being configured to cause, upon a change in a characteristic of the wound, a visible change in the control area on the basis of a chemical reaction that is due to the liquid or gaseous secretion so as to enable monitoring of the healing of the wound,
the control element comprising at least one receptor configured to selectively respond to one or more analytes selected from the group containing reactive oxygen species, amines, alcohols, thiols, aldehydes, ketones, diols, hydrogen peroxide, nitrogen oxide, amino acids, carboxylic acids, hydrogen sulfide, and mercaptans.

2. Dressing material as claimed in claim 1, wherein the control element (120) is disposed in or on the basic dressing material (110) so that the healing of the wound can be monitored without removing the dressing material from the wound.

3. Dressing material as claimed in claim 1 or 2, wherein the control element (120) is configured to change a color of the control area upon a change in the characteristic of the wound.

4. Dressing material as claimed in any of claims 1 to 3, wherein the control area of the control element (120) is a side of the control element (120) which faces the wound, and wherein the control element (120) is transparent, so that the control area is visible once the dressing material has been applied to the wound.

5. Dressing material as claimed in any of claims 1 to 4, wherein the control element comprises indicator dies for detecting reactive oxygen species, alcohols, diols, carboxylic acids, aldehydes, ketones, amino acids, hydrogen peroxide, nitrogen oxide, amines, thiols, mercaptans, carboxylic acids, and/or hydrogen sulfide.

6. Dressing material as claimed in any of claims 1 to 5, wherein part of the basic dressing material (110) is disposed between the control element (120) and the wound once the dressing material has been applied to the wound.

7. Dressing material as claimed in any of claims 1 to 5, further comprising an intermediate layer (230) disposed between the control element (120) and the wound, the intermediate layer (230) being configured to transport the liquid or gaseous secretion from the wound to the control element (120).

8. Dressing material as claimed in any of claims 1 to 7, wherein the control element (120) consists of a flexible material so as to adapt to a shape of a surface to which the dressing material is applied.

9. Dressing material as claimed in any of claims 1 to 8, wherein the visible change concerns part of a total area of the control area, the part in question of the total area having a size that is proportional to an area of the wound wherein which the characteristic of the wound changes, or wherein a site of the part in question of the total area is based on a site of the wound where the characteristic of the wound changes.

10. Dressing material as claimed in any of claims 1 to 9, wherein the liquid or gaseous secretion is a wound fluid of the wound.

11. Dressing material as claimed in any of claims 1 to 10, wherein the change in the characteristic of the wound corresponds to a change in a characteristic of a wound fluid of the wound.

12. Dressing material as claimed in any of claims 1 to 11, wherein the one or more receptors are introduced into porous structures, the porous structures being adapted to allow only analytes of specific sizes and structures to enter.

13. Plaster including a dressing material as claimed in any of claims 1 to 11.

14. Method (600) for detecting a change in a characteristic of a wound, comprising:
applying (610) a dressing material to a wound, the dressing material including a basic dressing material and a control element, the control element being disposed in or on the basic dressing material so that a control area of the control element is visible and that a liquid or gaseous secretion of a wound is in contact with the control element, wherein an ion-impermeable matrix is disposed between the wound and the control element, the control element being configured to cause, upon a change in a characteristic of the wound, a visible change in the control area on the basis of a chemical reaction; and
monitoring (620) the control area so as to detect a visible change in the control area and to thereby detect a change in a characteristic of the wound,
the control element comprising at least one receptor configured to selectively respond to one or more analytes selected from the group containing reactive oxygen species, amines, alcohols, thiols, aldehydes, ketones, diols, hydrogen peroxide, nitrogen oxide, amino acids, carboxylic acids, hydrogen sulfide, and mercaptans.

15. Method of producing a dressing material, comprising:
disposing a control element in or on a basic dressing material so that a control area of the control element is visible and that a liquid or gaseous secretion of a wound is in contact with the control element once the dressing material has been applied to the wound, and so that an ion-impermeable matrix is disposed between the wound and the control element once the dressing material has been applied to the wound, the control element being configured to cause a visible change in the control area on the basis of a chemical reaction upon a change in a characteristic of the wound so as enable monitoring of the healing of the wound, wherein the control element comprises at least one receptor configured to selectively respond to one or more analytes selected from the group containing reactive oxygen species, amines, alcohols, thiols, aldehydes, ketones, diols, hydrogen peroxide, nitrogen oxide, amino acids, carboxylic acids, hydrogen sulfide, and mercaptans.

## Revendications

1. Matériau de pansement (100, 200, 300, 400, 500) pour surveiller une cicatrisation de plaie, aux caractéristiques suivantes:
un matériau de pansement de base (110);
un élément de contrôle (120) disposé dans ou sur le matériau de pansement de base (110) de sorte que soit visible une surface de contrôle de l'élément de contrôle (120), et qu'une sécrétion liquide ou gazeuse d'une plaie soit en contact avec l'élément de contrôle lorsque le matériau de pansement est appliqué sur la plaie; et
une matrice imperméable aux ions qui est disposée entre la plaie et l'élément de contrôle lorsque le matériau de pansement est appliqué sur la plaie,
dans lequel l'élément de contrôle (120) est conçu pour provoquer, en cas de variation d'une propriété de la plaie, sur base d'une réaction chimique provoquée par la sécrétion liquide ou gazeuse, une modification visible de la surface de contrôle, pour permettre la surveillance de la cicatrisation de la plaie,
dans lequel l'élément de contrôle présente au moins un récepteur qui est conçu pour réagir sélectivement à un ou plusieurs analytes qui sont sélectionnés parmi le groupe contenant des espèces réactives de l'oxygène, des amines, des alcools, des thiols, des aldéhydes, des cétones, des diols, du peroxyde d'hydrogène, de l'oxyde d'azote, des acides aminés, des acides carboxyliques, du sulfure d'hydrogène et des mercaptans.

2. Matériau de pansement selon la revendication 1, dans lequel l'élément de contrôle (120) est disposé dans ou sur le matériau de pansement de base (110), de sorte que la cicatrisation de la plaie puisse être surveillée sans devoir retirer le matériau de pansement de la plaie.

3. Matériau de pansement selon la revendication 1 ou 2, dans lequel l'élément de contrôle (120) est conçu pour modifier, en cas de variation de la propriété de la plaie, une couleur de la surface de contrôle.

4. Matériau de pansement selon l'une des revendications 1 à 3, dans lequel la surface de contrôle de l'élément de contrôle (120) est une face de l'élément de contrôle (120) faisant face à la plaie, et dans lequel l'élément de contrôle (120) est transparent, de sorte que la surface de contrôle soit visible lorsque le matériau de pansement est appliqué sur la plaie.

5. Matériau de pansement selon l'une des revendications 1 à 4, dans lequel l'élément de contrôle présente des colorants indicateurs pour la détection d'espèces réactives de l'oxygène, des alcools, des diols, des acides carboxyliques, des aldéhydes, des cétones, des acides aminés, du peroxyde d'hydrogène, de l'oxyde d'azote, des amines, des thiols, des mercaptans, des acides carboxyliques et/ou du sulfure d'hydrogène.

6. Matériau de pansement selon l'une des revendications 1 à 5, dans lequel une partie du matériau de pansement de base (110) est disposée entre l'élément de contrôle (120) et la plaie lorsque le matériau de pansement est appliqué sur la plaie.

7. Matériau de pansement selon l'une des revendications 1 à 5, présentant par ailleurs une couche intermédiaire (230) qui est disposée entre l'élément de contrôle (120) et la plaie, dans lequel la couche intermédiaire (230) est conçue pour transporter la sécrétion liquide ou gazeuse de la plaie vers l'élément de contrôle (120).

8. Matériau de pansement selon l'une des revendications 1 à 7, dans lequel l'élément de contrôle (120) est réalisé en un matériau souple, pour s'adapter à une forme d'une surface sur laquelle est appliqué le matériau de pansement.

9. Matériau de pansement selon l'une des revendications 1 à 8, dans lequel la variation visible concerne une partie d'une superficie totale de la surface de contrôle, dans lequel une grandeur de la partie concernée de la superficie totale est proportionnelle à une surface de la plaie dans laquelle varie la propriété de la plaie, ou dans lequel un endroit de la partie concernée de la superficie totale se base sur un endroit de la plaie auquel varie la propriété de la plaie.

10. Matériau de pansement selon l'une des revendications 1 à 9, dans lequel la sécrétion liquide ou gazeuse est un fluide de la plaie.

11. Matériau de pansement selon l'une des revendications 1 à 10, dans lequel la variation de la propriété de la plaie correspond à une variation d'une propriété d'un fluide de la plaie.

12. Matériau de pansement selon l'une des revendications 1 à 11, dans lequel le ou les récepteurs sont incorporés dans des structures poreuses, dans lequel les structures poreuses sont adaptées pour ne laisser pénétrer que des analytes de grandeur et de structure déterminées.

13. Plâtre comportant un matériau de pansement selon l'une des revendications 1 à 11.

14. Procédé (600) de détection d'une variation d'une propriété d'une plaie, aux étapes suivantes consistant à:
appliquer (610) un matériau de pansement sur une plaie, le matériau de pansement comportant un matériau de pansement de base et un élément de contrôle, l'élément de contrôle étant disposé dans ou sur le matériau de pansement de base, de sorte que soit visible une surface de contrôle de l'élément de contrôle et
qu'une sécrétion liquide ou gazeuse d'une plaie soit en contact avec l'élément de contrôle, une matrice imperméable aux ions étant disposée entre la plaie et l'élément de contrôle, l'élément de contrôle étant conçu pour provoquer, en cas de variation d'une propriété de la plaie, sur base d'une réaction chimique, une modification visible de la surface de contrôle; et
surveiller (620) la surface de contrôle, pour détecter une variation visible de la surface de contrôle et, de ce fait, une variation d'une propriété de la plaie,
dans lequel l'élément de contrôle présente au moins un récepteur qui est conçu pour réagir sélectivement à un ou plusieurs analytes qui sont sélectionnés parmi le groupe contenant des espèces réactives de l'oxygène, des amines, des alcools, des thiols, des aldéhydes, des cétones, des diols, du peroxyde d'hydrogène, de l'oxyde d'azote, des acides aminés, des acides carboxyliques, du sulfure d'hydrogène et des mercaptans.

15. Procédé de fabrication d'un matériau de pansement, à l'étape suivante consistant à:
disposer un élément de contrôle dans ou sur un matériau de pansement de base, de sorte qu'une surface de contrôle de l'élément de contrôle soit visible et qu'une sécrétion liquide ou gazeuse d'une plaie soit en contact avec l'élément de contrôle lorsque le matériau de pansement est appliqué sur la plaie, et de sorte qu'une matrice imperméable aux ions soit disposée entre la plaie et l'élément de contrôle lorsque le matériau de pansement est appliqué sur la plaie, l'élément de contrôle étant conçu pour provoquer, en cas de variation d'une propriété de la plaie, sur base d'une réaction chimique, une variation visible de la surface de contrôle pour permettre une surveillance de la cicatrisation de la plaie, l'élément de contrôle présentant au moins un récepteur qui est conçu pour réagir sélectivement à un ou plusieurs analytes qui sont sélectionnés parmi le groupe contenant des espèces réactives de l'oxygène, des amines, des alcools, des thiols, des aldéhydes, des cétones, des diols, du peroxyde d'hydrogène, de l'oxyde d'azote, des acides aminés, des acides carboxyliques, du sulfure d'hydrogène et des mercaptans.
